# EUROPEAN PATENT APPLICATION

(11) **EP 2 896 352 A1**
(43) Date of publication of application: **22.07.2015**
(21) Application number: 13877296.7
(22) Date of filing: 22.11.2013
(51) Int. Cl.: A61B 1/00, G02B 23/24

(54) **ENDOSCOPE**

(30) Priority: 08.03.2013 JP 2013047117
(71) Applicant: OLYMPUS CORPORATION, Shibuya-ku Tokyo 151-0072 (JP)
(72) Inventor: KUDO Ryota, Tokyo 151-0072 (JP); OUCHI Naoya, Tokyo 151-0072 (JP)
(74) Representative: Schicker, Silvia
(86) International application number: PCT/JP2013/081505
(87) International publication number: WO 2014/136326

(57) **Abstract**

An endoscope 1 of the invention includes: an operation portion 3; an insertion portion 2; a treatment instrument insertion channel 17 through which an elongated treatment instrument 30 is inserted; a treatment instrument raising base 21; an observation window 25 arranged closer to the operation portion 3 than the treatment instrument raising base 21 in a longitudinal direction of the insertion portion 2 at the distal end portion 5 of the insertion portion 2; and a restricting surface 31A of a restricting portion 31 configured to contact the treatment instrument 30 which is protruded by being directed by the treatment instrument raising base 21 when the treatment instrument raising base 21 is inclined and to restrict an angle of the treatment instrument protruding outside the distal end portion 5, wherein a line 01 connecting the treatment instrument raising base 21 and the observation window 25 is substantially parallel to a longitudinal axis O in a direction in which the insertion portion 2 is inserted, and the restricting surface 31A is provided so as to be located on a line segment connecting the treatment instrument raising base 21 and the observation window 25 when the observation window 25 is viewed from right in front.

## Description

### Technical Field

The present invention relates to an endoscope that includes an insertion portion, a treatment instrument insertion channel through which a treatment instrument is inserted, and a treatment instrument raising base that changes a protruding direction of the treatment instrument.

### Background Art

In recent years, endoscopes have been widely used in medical fields. Some endoscopes for use in medical fields are provided with a treatment instrument insertion channel through which a treatment instrument can be inserted so that treatment such as medical attention can be performed using the treatment instrument.

For example, lateral-view endoscopes used for ERCP (Endoscopic Retrograde Cholangio-Pancreatography) include, at the distal end portion thereof, a treatment instrument raising base that changes a protruding direction of the treatment instrument.

As a conventional endoscope of this type, for example, Japanese Patent Application Laid-Open Publication No. 6-254037 discloses an endoscope cover with a channel.

The endoscope cover with a channel recited in the Japanese Patent Application Laid-Open Publication No. 6-254037 includes, on a plane surface formed on a side portion in the vicinity of a distal end of a distal end constituting portion, an observation optical system which configures an observation window and is disposed on the distal end side of the plane surface and an illumination optical system which configures an illumination window and which is disposed on both sides of the observation optical system, such that the observation optical system and the illumination optical systems align in the direction vertical to the axis direction of the insertion portion, and also includes at the rear of the observation optical system along the axis direction, a channel opening portion which communicates with a treatment instrument insertion channel provided inside the cover, and a treatment instrument raising base disposed inside the channel opening portion.

In addition, as a prior art similar to the one disclosed in the Japanese Patent Application Laid-Open Publication No. 6-254037, Japanese Patent Application Laid-Open Publication No. 63-286132 discloses a rigid endoscope in which a slope-shaped opening portion that opens on a sheath opening portion side is formed at a distal end of a distal end portion main body, a cover glass and an objective lens system which is opposed to the cover glass and set in an oblique direction such that the observation field-of-view direction has a predetermined angle with respect to the axis direction of the insertion portion are housed in the opening portion, and a treatment instrument raising device is provided at a position which is at the rear of the cover glass and the objective lens system in the axis direction of the insertion portion and which is on the side portion of the opening of the sheath.

In a case where the endoscope having the treatment instrument raising base is configured such that a longitudinal axis of the treatment instrument which is protruded along the treatment instrument raising base and an optical axis of the objective optical system do not coincide with each other when viewed from the distal end side, when it is attempted to insert the treatment instrument such as a tube into the duodenal papilla in the ERCP, for example, an endoscopic image in which an axis of the bile duct and the longitudinal axis of the treatment instrument do not coincide with each other is displayed. Therefore, in the endoscope having such a configuration, it is difficult to insert the treatment instrument into the duodenal papilla.

Therefore, as a countermeasure, it is considered to arrange the treatment instrument raising base and the objective optical system to be substantially parallel to a longitudinal axis of the insertion portion and on a straight line, as shown in Japanese Patent Application Laid-Open Publication No. 6-254037 and Japanese Patent Application Laid-Open Publication No. 63-286132.

However, in Japanese Patent Application Laid-Open Publication No. 6-254037 having such a configuration, when the treatment instrument raising base is raised, an endoscopic image in which the treatment instrument itself largely covers a field of view of the objective optical system is displayed. Therefore, such an endoscope has a fear that it is difficult for a surgeon to confirm a treatment target to be treated by the treatment instrument and to perform the treatment.

Further, since the rigid endoscope described in Japanese Patent Application Laid-Open Publication No. 63-286132 has a field-of-view range of the objective optical system in an oblique direction forward of the distal end portion of the insertion portion, it is impossible to pick up an image of the subject to be treated in a direction opposite to the insertion direction of the insertion portion, and thus it is impossible to perform endoscopy and medical attention such as the ERCP in which the treatment instrument, which is protruded in the direction opposite to the insertion direction of the insertion portion, is inserted into the duodenal papilla.

The present invention has been made in view of the above problems and an object of the present invention is to provide an endoscope capable of easily performing procedure by making an axis of a site of a treatment target and a longitudinal axis of a treatment instrument coincide with each other so that a field of view at a part to be treated is not obstructed by the treatment instrument.

### Disclosure of Invention

### Means for Solving the Problem

An endoscope according to one aspect of the present invention includes: an operation portion; an insertion portion to be inserted in a lumen, the insertion portion being connected to the operation portion; a treatment instrument insertion channel through which an elongated treatment instrument is inserted, the treatment instrument insertion channel opening at a distal end portion of the insertion portion and being extended in the insertion portion from a proximal end side of the insertion portion to a part where the treatment insertion channel opens; a treatment instrument raising base pivotally supported to be rotatable with respect to the distal end portion of the insertion portion, configured to control an inclination angle by an operation of the operation portion, and arranged to face the part where the treatment instrument insertion channel opens at the distal end portion of the insertion portion; an objective optical system configured to obtain an image of a subject to be examined in the lumen and arranged closer to the operation portion than the treatment instrument raising base in a longitudinal direction of the insertion portion at the distal end portion of the insertion portion; and a restricting portion configured to contact the treatment instrument which is protruded by being directed by the treatment instrument raising base when the treatment instrument raising base is inclined and to restrict an angle of the treatment instrument protruding outside the distal end portion of the insertion portion, wherein a line connecting the treatment instrument raising base and the objective optical system is substantially parallel to a longitudinal axis in a direction in which the insertion portion is inserted, and the restricting portion is provided so as to be located on a line segment connecting the treatment instrument raising base and the objective optical system when the objective optical system is viewed from right in front.

### Brief Description of the Drawings

FIG. 1 is a perspective view showing an overall configuration of an endoscope according to a first embodiment of the present invention.
FIG. 2 is a perspective view showing a configuration of a distal end portion of the endoscope in FIG. 1.
FIG. 3 is a top view of the distal end portion of the endoscope in FIG. 1.
FIG. 4 is a cross-sectional view taken along the IV-IV line in FIG. 3, which shows a state where the treatment instrument raising base has been completely raised.
FIG. 5 is a cross-sectional view of the distal end portion, for explaining a relationship between the treatment instrument raising base and a restricting surface of a distal end rigid portion.
FIG. 6 is a cross-sectional view of a distal end portion of a conventional endoscope, which shows a state where the treatment instrument raising base has been completely raised.
FIG. 7 is a view showing an endoscopic image obtained by the conventional endoscope in FIG. 6.
FIG. 8 is a view showing an endoscopic image obtained by the endoscope according to the present embodiment.
FIG. 9 is an illustration diagram in a case where treatment is performed on a gastric angle of a stomach using the endoscope according to the present embodiment.
FIG. 10A is a view illustrating a configuration of a restricting surface provided at a restricting portion as a member different from the distal end rigid portion, which is viewed from a front direction in the longitudinal axis direction of an insertion portion 2.
FIG. 10B is a view illustrating the configuration of the restricting surface provided at the restricting portion as a member different from the distal end rigid portion, which is viewed from a direction perpendicular to the restricting surface.
FIG. 10C is a view illustrating the configuration of the restricting surface provided at the restricting portion as a member different from the distal end rigid portion, and is a side view of the restricting portion having the restricting surface.
FIG. 11A is a view illustrating a configuration of a restricting surface according to a modified example 1, which is viewed from the front direction in the longitudinal axis direction of the insertion portion 2.
FIG. 11B is a view illustrating the configuration of the restricting surface according to the modified example 1, which is viewed from the direction perpendicular to the restricting surface.
FIG. 11C is a view illustrating the configuration of the restricting surface according to the modified example 1, and is a side view of the restricting portion including the restricting surface.
FIG. 12A is a view illustrating a configuration of a restricting surface according to a modified example 2, which is viewed from the front direction in the longitudinal axis direction of the insertion portion 2.
FIG. 12B is a view illustrating the configuration of the restricting surface according to the modified example 2, which is viewed from the direction perpendicular to the restricting surface.
FIG. 12C is a view illustrating the configuration of the restricting surface according to the modified example 2, and is a side view of the restricting portion including the restricting surface.
FIG. 13A is a view illustrating another configuration of the restricting surface according to the modified example 2, which is viewed from the front direction in the longitudinal axis direction of the insertion portion 2.
FIG. 13B is a view illustrating the other configuration of the restricting surface according to the modified example 2, which is viewed from the direction perpendicular to the restricting surface.
FIG. 13C is a view illustrating the other configuration of the restricting surface according to the modified example 2, and is a side view of the restricting portion including the restricting surface.
FIG. 14 is a cross-sectional view of a distal end portion, which shows a configuration of an illumination optical system according to a modified example 3.
FIG. 15 is a top view of the distal end portion, which shows a configuration of an illumination optical system according to a modified example 4.
FIG. 16 is a cross-sectional view taken along the XV-XV line in FIG. 15.
FIG. 17 is a top view of a distal end portion of an endoscope according to a second embodiment of the present invention.

### Best Mode for Carrying Out the Invention

Hereinafter, detailed description will be made on embodiments of the present invention with reference to drawings.

### (First Embodiment)

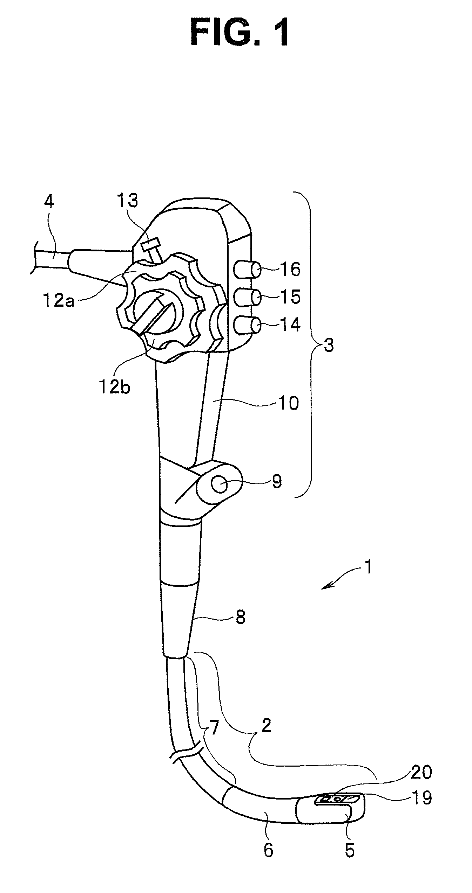

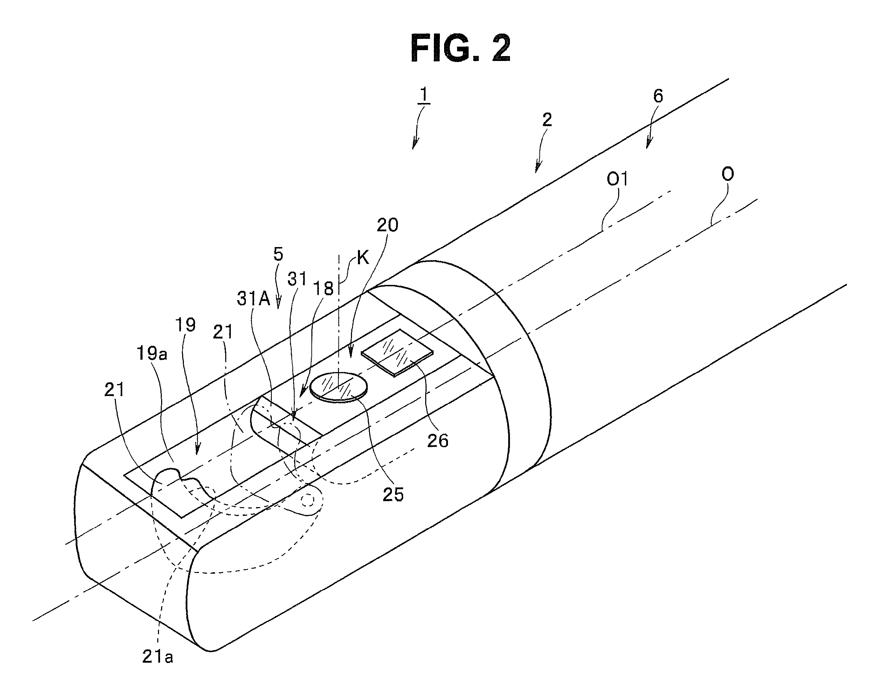

FIG. 1 is a perspective view showing an overall configuration of an endoscope according to a first embodiment of the present invention, and FIG. 2 is a perspective view showing a configuration of a distal end portion of the endoscope in FIG. 1.

As shown in FIGS. 1 and 2, an endoscope 1 according to the present embodiment is configured as a lateral-view endoscope, for example, and includes an insertion portion 2 and an operation portion 3. The operation portion 3 is connected with a universal cord 4 which includes inside thereof a light guide fiber and the like.

The insertion portion 2 is configured by including, in the following order from the distal end, a distal end portion 5, a bending portion 6, and a flexible tube portion 7 which are provided in a linked manner.

The operation portion 3 includes a forceps port 9 disposed on a side to which the proximal end of a bend preventing portion 8 for the insertion portion 2 is connected, a grasping portion 10 as a middle portion, two bending operation knobs, i.e., an up-down bending operation knob 12a and a right-left bending operation knob 12b which are provided at the upper portion side of the grasping potion 10, a raising operation lever 13 for performing raising operation of a treatment instrument raising base 21 to be described later, an air/water feeding control button 14, a suction control button 15, and an image recording button 16.

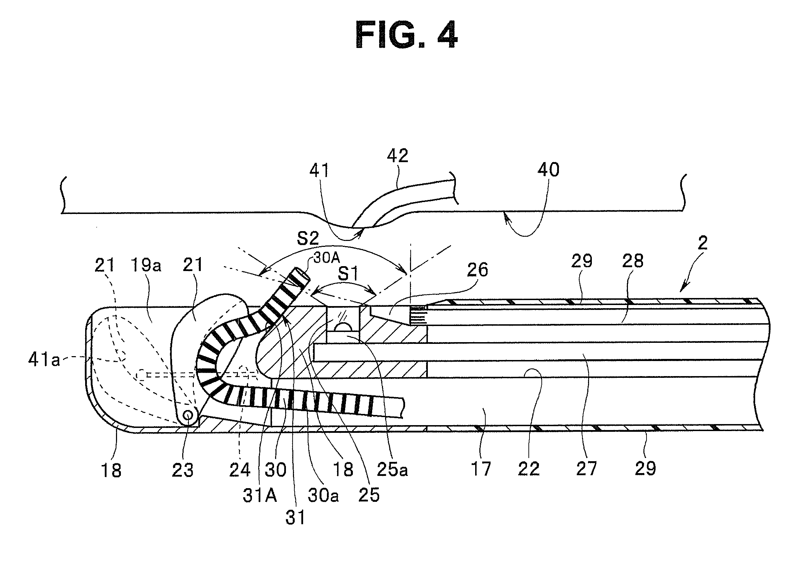

Note that the forceps port 9 is communicated with a channel opening portion 19 through a treatment instrument insertion channel 17 disposed in the insertion portion 2 (see FIG. 4).

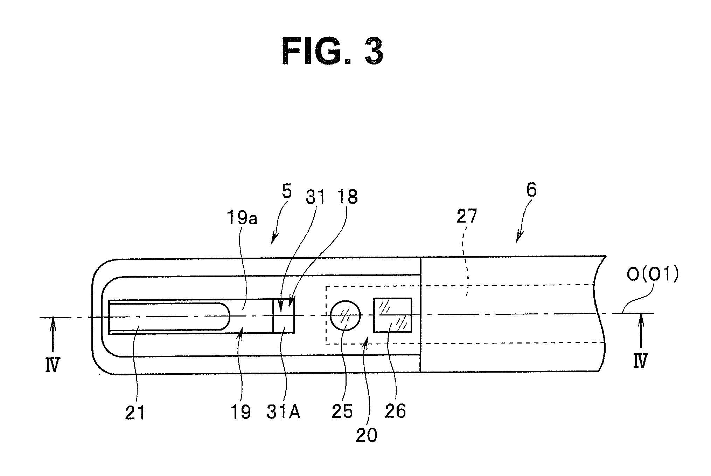

Next, specific configuration of the distal end portion 5 will be described with reference to FIGS. 1 to 4. FIG. 3 is a top view of the distal end portion of the endoscope 1 in FIG. 1, and FIG. 4 is a cross-sectional view taken along the IV-IV line in FIG. 3, which shows a state where the treatment instrument raising base has been completely raised. Note that FIG. 4 schematically shows constituent members in the distal end portion.

The distal end portion 5 shown in FIG. 1 is provided with the channel opening portion 19 and an observation opening portion 20.

As shown in FIG. 2, the treatment instrument raising base 21 is disposed in the channel opening portion 19.

The observation opening portion 20 is provided with a transparent cover glass, not shown, and inside the distal end portion 5, an observation window 25 and an illumination window 26 are disposed in this order from the distal end side along an axis O1 which is parallel to the central axis (also referred to as a longitudinal axis along which the insertion portion 2 is inserted) O of the insertion portion 2, so as to enable an observation of a site to be examined by irradiating the site to be examined with illumination light.

Detailed description will be made on the configuration of the distal end portion 5 having the treatment instrument raising base 21. As shown in FIG. 2 and FIG. 4, the distal end portion 5 includes a distal end rigid portion 18.

The distal end rigid portion 18 is made of a rigid resin or the like, and provided with a channel distal end space portion 19a which is communicated with the treatment instrument insertion channel 17. The distal end portion of the channel distal end space portion 19a opens outside through the channel opening portion 19. Note that the distal end portion 5 is configured such that the outer circumference from the rear end side to the proximal end side of the distal end rigid portion 18 is covered with an outer cover 29.

In addition, the distal end portion of a channel tube 22 which configures the treatment instrument insertion channel 17 is inserted in a hole which is communicated with the channel distal end space portion 19a of the distal end rigid portion 18 of the distal end portion 5. That is, the distal end portion of the channel tube 22 is connected to the distal end rigid portion 18 and communicated with the channel distal end space portion 19a provided at the distal end rigid portion 18.

The treatment instrument raising base 21 that raises a treatment instrument 30 inserted through the treatment instrument insertion channel 17 is rotatably arranged in the channel distal end space portion 19a. The treatment instrument raising base 21 is arranged so as to face the part where the treatment instrument insertion channel 17 opens, and the proximal end portion of the treatment instrument raising base is pivotally supported by a rotational axis 23 such that the treatment instrument raising base is rotationally movable in the channel distal end space portion 19a with the proximal end portion as a center. Furthermore, the treatment instrument raising base 21 is connected with a raising wire 24 for raising and falling back the treatment instrument raising base 21.

The raising wire 24 passes through a wire tube (not shown) connected to the distal end rigid portion 18, and extends to the operation portion 3 side through the inside of the insertion portion 2. The proximal end of the raising wire 24 is connected to the raising operation lever 13. Therefore, an inclination angle of the treatment instrument raising base 21 is controlled by the raising operation lever 13.

That is, with the above-described configuration, one raising wire 24 connected to the treatment instrument raising base 21 is pulled or pushed out with the raising operation lever 13 of the operation portion 3, to perform operation to advance or retract the raising wire 24 in the direction of central axis O, thereby causing the treatment instrument raising base 21 contacting the side surface of the treatment instrument 30 protruded from the channel opening portion 19 of the distal end portion 5 to rotationally move, to enable the protruding direction of the treatment instrument 30 to be changed.

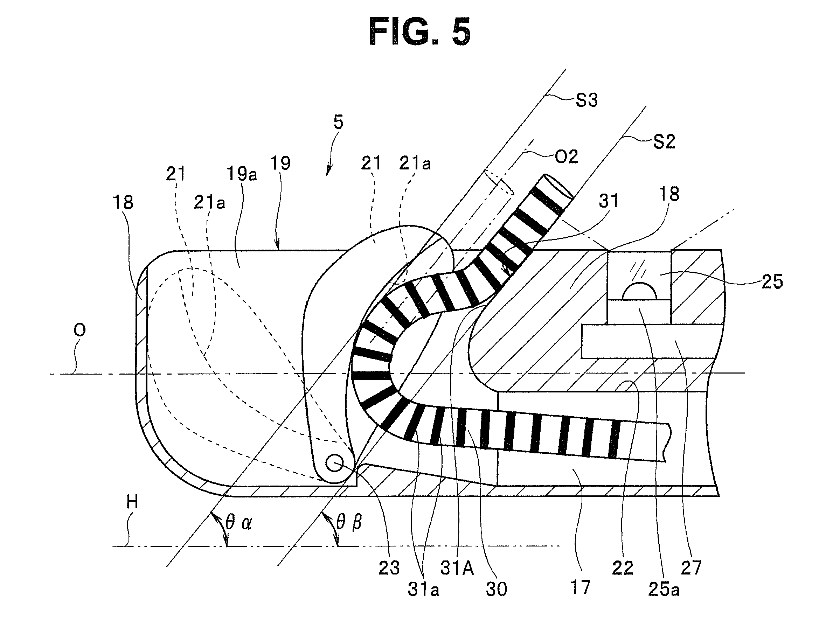

Note that the treatment instrument 30 shown in FIGS. 4 and 5 is a treatment instrument for performing treatment such as ERCP, for example, and is a tube (cannula) which is called as a contrast tube to be inserted into the duodenal papilla. On the outer circumferential surface from the distal end portion to the proximal end portion of the treatment instrument 30, stripe marks 30a as indications of an insertion amount, an insertion position and the like are provided at equal intervals. It is needless to say that the treatment instrument 30 is not limited to the contrast tube, but is used in accordance with procedure to be performed.

The observation window 25 of the observation opening portion 20 is arranged at the distal end portion 5 so as to be located closer to the operation portion in the longitudinal direction of the insertion portion 2 than the treatment instrument raising base 21. The observation window 18 is an optical system lens which configures a part of the objective optical system. As shown in FIG. 4, a solid-state image pickup device 25a such as CCD is disposed at the lower portion of the observation window 18.

The solid-state image pickup device 25a is fixed to a substrate, not shown, arranged on the distal end side of an image pickup cable 27 inserted in the insertion portion 2, and electrically connected to the image pickup cable 27 through the substrate, not shown.

Note that the observation window 18 which is a part of the objective optical system has a field-of-view range S1 as shown in FIG. 4, and configured to acquire an image of a subject to be examined in a lumen within the field-of-view range S1.

As shown in FIGS. 3 and 4, the illumination window 26 which configures the illumination optical system is disposed on the operation portion side which is rear side of the observation window 25 on the axis O1. The illumination window 26 emits illumination light guided from a light emitter through the light guide 28, to illuminate the subject to be examined in the lumen. The illumination window 26 is a transparent optical system member having an irradiation range S2 shown in FIG. 4, and illuminates the subject to be examined in the lumen within the irradiation range S2.

As shown in FIGS. 3 and 4, in the present embodiment, the distal end portion 5 of the endoscope 1 includes a restricting portion 31 which contacts the treatment instrument 30 directed by the treatment instrument raising base 21 to be protruded, when the treatment instrument raising base 21 is inclined, and restricts an angle at which the treatment instrument 30 is protruded outside the distal end portion 5 of the insertion portion 2.

In addition, at the distal end portion 5, the axis O1 which is a line connecting the treatment instrument raising base 21 and the observation window 25 is substantially parallel to the longitudinal axis (central axis) O which is along the insertion direction of the insertion portion 2 (see FIG. 3). Furthermore, the restricting portion 31 includes a restricting surface 31 A, and the restricting surface 31 A is provided so as to be positioned on the line segment connecting the treatment instrument raising base 21 and the observation window 25 when the observation window 25 is viewed from right in front (see FIG. 3).

More specifically, as shown in FIG. 2, the observation window 25 is configured such that the optical axis K faces the direction perpendicular to the longitudinal axis (central axis) O which is along the insertion direction of the insertion portion 2, for example. The restricting surface 31A of the restricting portion 31 directs the treatment instrument 30 at an angle at which the outer surface of the treatment instrument 30 does not obstruct the distal end field of view toward which the treatment instrument 30 extends in the field-of-view range S1 of the observation window 25 (see FIG. 4).

In the present embodiment, the restricting surface 31 A of the restricting portion 31 has a predetermined area which contacts the treatment instrument 30 and is configured to restrict the angle of the treatment instrument 30. As shown in FIG 4, the restricting surface 31A of the restricting portion 31 is provided integrally with the distal end rigid portion 18, and is a plane formed on the upper side of the distal end rigid portion 18 at the position opposed to the treatment instrument raising base 21.

Note that the restricting surface 31A of the restricting portion 31 does not have to be provided integrally with the distal end rigid portion 18, but may be configured as a separated member.

The optical axis of the observation window 25 is not limited to the direction perpendicular to the longitudinal axis O, but may be inclined at a predetermined angle, that is, so as not to be parallel to the longitudinal axis O.

Description will be made on the arrangement relationship between the treatment instrument raising base 21 and the restricting surface 31A of the restricting portion 31 with reference to FIG. 5. FIG. 5 is a cross-sectional view of the distal end portion, for describing the relationship between the treatment instrument raising base and the restricting surface of the distal end rigid portion.

In the present embodiment, as shown in FIG. 5, when the treatment instrument raising base 21 is maximally inclined, if it is supposed that the angle of the central axis 02 of the treatment instrument 30 with respect to the longitudinal axis (central axis) O which is along the insertion direction of the insertion portion 2 is θα and the angle of the restricting surface 31A with respect to the longitudinal axis O is θβ, the angle θα is equal to or smaller than the angle θβ of the restricting surface 31A. That is, the endoscope 1 according to the present embodiment is configured to have the relationship of θα ≤ θβ. In other words, the treatment instrument raising base 21 is capable of inclining the treatment instrument 30 largely until the angle of the central axis 02 of the treatment instrument 30 with respect to the longitudinal axis O becomes the angle θα. However, the restricting surface 31 A is provided, thereby restricting the angle of the central axis 02 of the treatment instrument 30 with respect to the longitudinal axis O to an angle equal to or smaller than the angle θβ which is larger than the angle θα. That is, the restricting surface is capable of allowing the angle at which the treatment instrument 30 is inclined by the treatment instrument raising base 21 to be slightly smaller than the angle θβ. Note that the angle θα and the angle θβ may be changed appropriately as needed. In FIG. 5, an extension line S2 is an extension line of the restricting surface 31A, an extension line S3 is an extension line parallel to the central axis 02 of the treatment instrument 30, and a virtual line H is a line parallel to the longitudinal axis O.

With such a configuration, as shown in FIG. 4, the restricting portion 31 is capable of directing the treatment instrument 30 at angle at which the outer surface of the treatment instrument 30 does not obstruct the distal end field of view toward which the treatment instrument 30 extends in the field-of-view range S1 of the observation window 25.

Note that, in the present embodiment, the illumination window 26 is provided such that the center of the illumination window 26 overlaps on the straight line (on the axis O1) connecting the treatment instrument raising base 21 and the observation window 25 when the illumination window 26 is viewed from right in front.

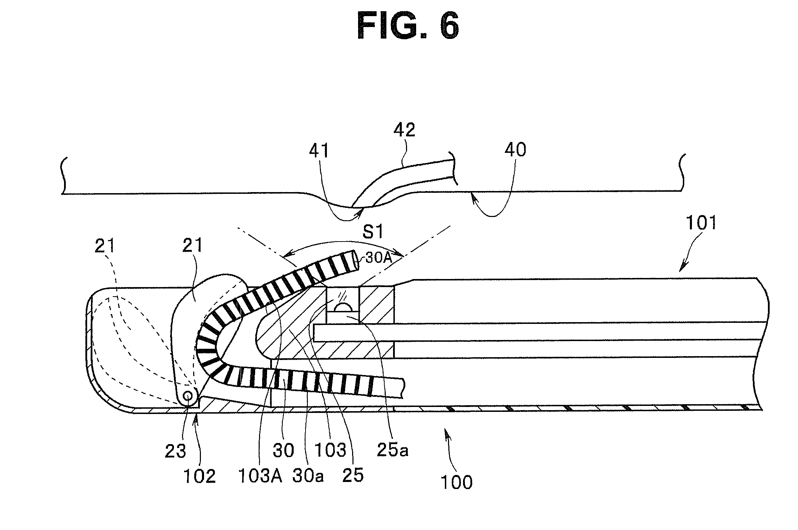

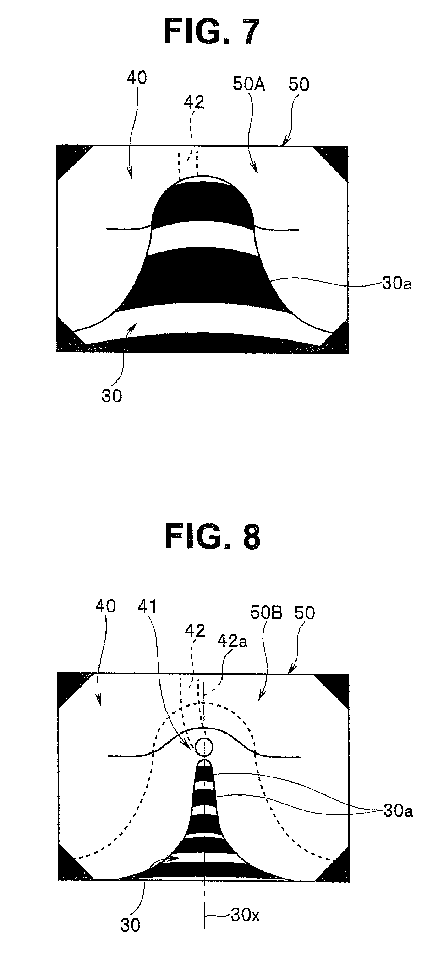

Next, description will be made on working of the restricting portion 31 of the endoscope according to the present embodiment with reference to FIGS. 4 to 8. FIG. 6 is a cross-sectional view of a distal end portion of a conventional endoscope, which shows a state where the treatment instrument raising base has been completely raised. FIG. 7 is a view showing an endoscopic image obtained by the conventional endoscope in FIG. 6. FIG. 8 is a view showing an endoscopic image obtained by the endoscope according to the present embodiment.

It is now supposed that a procedure, such as ERCP, of inserting the treatment instrument 30 into the duodenal papilla is performed by using the endoscope 1 according to the present embodiment. An operator inserts the insertion portion 2 of the endoscope 1 into the lumen of a patient, to advance the distal end portion 5 until the distal end portion reaches a position where an image of the duodenal papilla 41 of the duodenum 40 can be seen, and then places the distal end portion 5 at the position, as shown in FIG. 4.

In this case, as is known, the bile duct 42 in the duodenal papilla 41 is located in the insertion direction of the insertion portion 2. Therefore, the operator inserts the insertion portion 2 so as to place the distal end portion 5 at a position slightly deeper side of the duodenal papilla 41 while viewing an endoscopic image.

After that, the operator brings the treatment instrument 30, which has been inserted through the forceps port 9 and the treatment instrument insertion channel 17 in the insertion portion 2, into contact with a concave portion 21a formed on the side surface of the treatment instrument raising base 21 in the channel distal end space portion 19a and advances the treatment instrument 30 until the distal end portion 30A of the treatment instrument 30 protrudes upward from the channel opening portion 19.

Then, the operator operates the raising operation lever 13 of the operation portion 3 to pull the one raising wire 24 connected to the treatment instrument raising base 21, to thereby perform operation for moving the raising wire 24 in the direction opposite to the insertion direction of the insertion portion 2. With such an operation, the treatment instrument raising base 21 which contacts the side surface of the treatment instrument 30 protruded from the channel opening portion 19 of the distal end portion 5 moves rotationally, and thereby capable of changing the protruding direction of the treatment instrument 30.

As shown in FIG. 6, in the conventional endoscope, even if the treatment instrument raising base 21 is moved rotationally to change the protruding direction of the treatment instrument 30 protruded from the channel opening portion, and the treatment instrument is brought into contact with a part of the contacting surface 103A of the distal end rigid portion 103, the treatment instrument 30 itself enters into the field-of-view range S1 of the observation window 25.

As a result, as shown in FIG. 7, since the distal end portion 30A of the treatment instrument 30 is displayed so as to fill the entire screen of the monitor 50 which is connected to the endoscope 1 to display an endoscopic image, an endoscopic image 50A is displayed in which the duodenal papilla 41 into which the treatment instrument is to be inserted is hidden by the treatment instrument 30 and cannot be recognized. Therefore, the operator cannot smoothly insert the distal end portion 30A of the treatment instrument 40 into the bile duct 42 in the duodenal papilla 41 while viewing the endoscopic image 50A as described above.

In contrast, in the present embodiment, the treatment instrument 30 which is directed by the treatment instrument raising base 21 to be protruded contacts the restricting surface 31 A of the restricting portion 31 provided at the distal end portion 5 of the endoscope 1, and thereby the angle at which the treatment instrument is protruded outside the distal end portion 5 of the insertion portion 2 is restricted.

That is, as described above with reference to FIG. 5, the restricting surface 31 A of the restricting portion 31 is configured such that, when the treatment instrument raising base 21 is maximally inclined, the angle θα of the central axis 02 of the treatment instrument 30 with respect to the longitudinal axis (central axis) O which is along the insertion direction of the insertion portion 2 becomes equal to or smaller than the angle θβ of the restricting surface 31 A with respect to the longitudinal axis O.

Therefore, as shown in FIG. 4, the treatment instrument 30 is directed by the restricting surface 31 A of the restricting portion 31 at the angle at which the outer surface of the treatment instrument 30 does not obstruct the distal end field of view toward which the treatment instrument 30 extends in the field-of-view range S1 of the observation window 25, that is, the treatment instrument 30 is directed toward the duodenal papilla 41.

Furthermore, in the present embodiment, at the distal end portion 5, the line O1 which connects the treatment instrument raising base 21 and the observation window 25 is substantially parallel to the longitudinal axis (central axis) O which is along the insertion direction of the insertion portion 2 (see FIG. 3). Furthermore, the restricting surface 31A of the restricting portion 31 is arranged at the position where the center of the restricting surface overlaps on the line segment connecting the treatment instrument raising base 21 and the observation window 25 (see FIG. 3).

Therefore, the endoscopic image obtained when the treatment instrument 30 is directed toward the duodenal papilla 41 by using the restricting surface 31 A may be the endoscopic image 50B as shown in FIG. 8.

That is, as shown in FIG. 8, the configuration according to the present embodiment is capable of obtaining a field of view , i.e., the endoscopic image 50B in which the treatment instrument 30 itself does not widely overlap the duodenal papilla 41 as a treatment target, and capable of displaying the endoscopic image 50B on the monitor 50.

The configuration also enables the axis 30x in the longitudinal direction of the treatment instrument 30 to coincide with the axis (center) 42a of the bile duct 42 in the duodenal papilla 41 as a treatment target in the endoscopic image 50B.

Thus, in the endoscopic image 50B, the treatment instrument 30 is displayed from the lower direction of the screen so as not to widely overlap the duodenal papilla 41, and the axis 42a of the bile duct 42 in the duodenal papilla 41 as a treatment target coincides with the longitudinal axis 30x of the treatment instrument 30, which provides an endoscopic image with reference to which the operator can easily insert the distal end portion 30a of the treatment instrument 30 into the bile duct 42.

Therefore, the operator is easily capable of aligning the position of the distal end portion 30A of the treatment instrument 30 with the bile duct 42 as a treatment target, while viewing the endoscopic image 50B shown in FIG. 8. As a result, the operator is capable of easily inserting the treatment instrument 30 into the bile duct 42 by gradually inserting the treatment instrument 30.

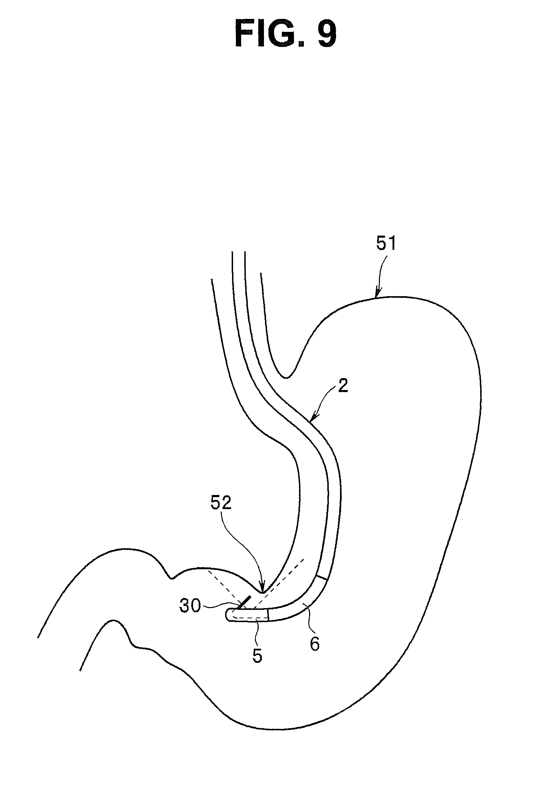

Note that description has been made on a medical procedure, such as ERCP, of inserting the treatment instrument 30 into the duodenal papilla 41 in the present embodiment. However, the present invention is not limited to such a medical procedure. For example, as shown in FIG. 9, a front-view type upper gastrointestinal endoscope is applicable also to the gastric angle 52 of the stomach 51 for which observation and treatment are difficult to perform. Such a front-view type upper gastrointestinal endoscope is capable of displaying on the monitor 50 an endoscopic image in which the treatment instrument 30 itself does not overlap the gastric angle 52 for which the treatment instrument performs treatment, and displaying the longitudinal axis of the treatment instrument 30 so as to align with the axis (center) of the gastric angle 52, thereby enabling treatment to be easily performed for the gastric angle 52 with the treatment instrument 30.

In addition, since the endoscope 1 according to the present embodiment is capable of accurately aligning the treatment instrument 30 with the target part, even in the case where the treatment target in the lumen is a part covered with folds, other than the gastric angle 51, the endoscope 1 is capable of effectively performing treatment.

Therefore, the first embodiment can provide the endoscope 1 which enables treatment to be easily performed by allowing the axis of the treatment target site to coincide with the longitudinal axis of the treatment instrument 30, without obstructing the field of view of the part to be treated with the treatment instrument 30.

In addition, in the present embodiment, the illumination window 26 is arranged at a position where the center of the illumination window 26 overlaps on the straight line (central axis O) connecting the treatment instrument raising base 21 and the observation window 25 when the illumination window 26 is viewed from right in front, so as to be located on the rear side of (closer to the operation portion than) the observation window 25. With such a configuration, the light guide 28 can be inserted into the insertion portion 2 without being bent so as to avoid the treatment instrument insertion channel 17. This enables the diameters of the insertion portion 2 and the distal end portion 5 to be reduced.

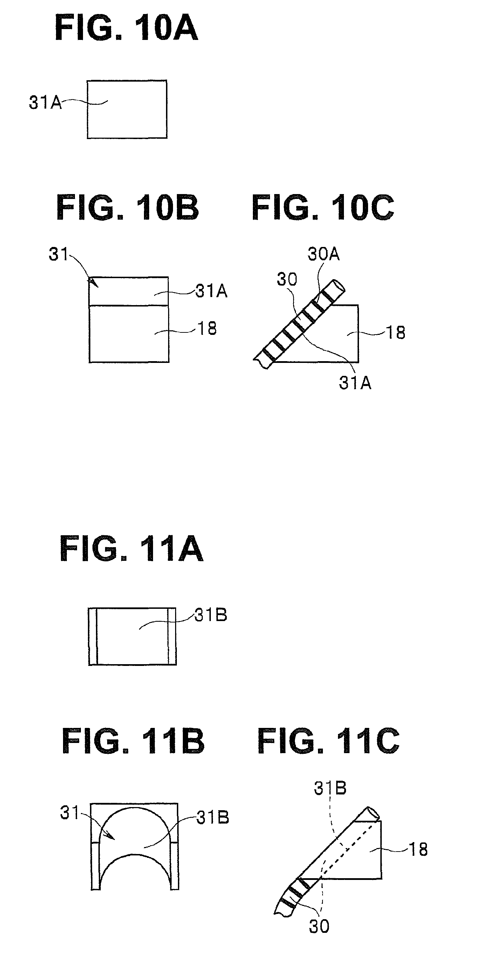

Note that the restricting surface 31A of the restricting portion 31 may be a plane formed at the restricting portion which is configured as a member different from the distal end rigid portion 18, as shown in FIGS. 10A, 10B and 10C. FIGS. 10A to 10C are views each illustrating the configuration of the restricting surface provided at the restricting portion as a member different from the distal end rigid portion. In FIGS. 10A to 10C, FIG. 10A is a view seen from a front direction in a longitudinal axis direction of an insertion portion 2, FIG. 10B is a view seen from the direction perpendicular to the restricting surface, and FIG. 10C is a side view of the restricting portion having the restricting surface.

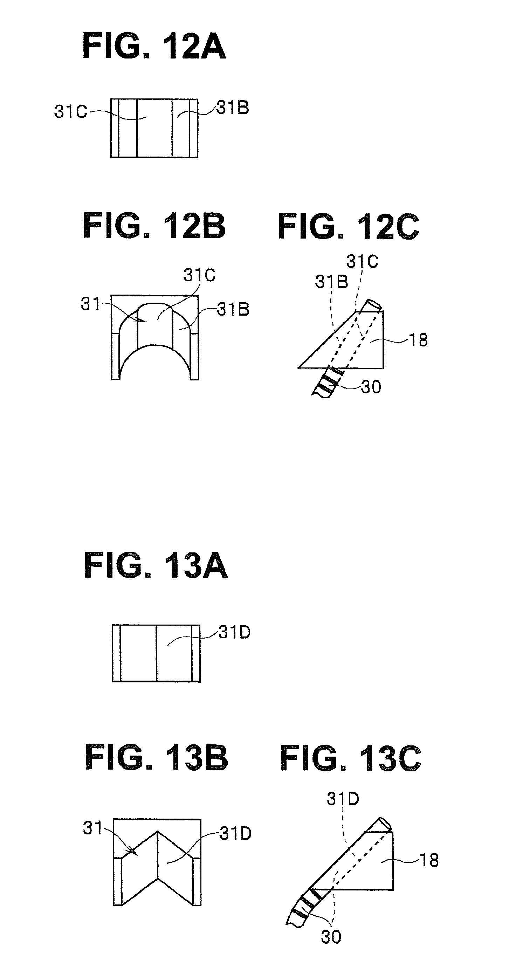

The restricting surface 31A of the restricting portion 31 according to the present embodiment may be configured as in modified examples 1 and 2 to be described later, for example. The modified examples 1, 2 are shown in FIGS. 11 and 12.

### (Modified Example 1)

FIGS. 11A to 11C are views each illustrating a configuration of the restricting surface according to the modified example 1. Note that FIG. 11A is a view seen from the front direction in the longitudinal axis direction of the insertion portion 2, FIG. 11B is a view seen from the direction perpendicular to the restricting surface, and FIG. 11C is a side view of the restricting portion having the restricting surface. In addition, the restricting portion 31 is configured as a member different from the distal end rigid portion 18.

In the first embodiment, the restricting surface 31 A of the restricting portion 31 is configured by a plane. On the other hand, the restricting surface 31B according to the modified example 1 is configured by including a groove for guiding the treatment instrument 30 so as to be substantially parallel to the longitudinal axis O which is along the insertion direction of the insertion portion 2, as shown in FIGS. 11A, 11B, and 11C. That is, the restricting surface 31B is configured as an arc surface provided in the concave portion of the groove.

Note that the arc shape of the restricting surface 31B just has to be formed in accordance with the diameter of the treatment instrument 30 to be required.

### (Modified Example 2)

FIGS. 12A to 12C are views each illustrating a configuration of the restricting surface according to the modified example 2. Note that FIG. 12A is a view seen from the front direction in the longitudinal axis direction of the insertion portion 2, FIG. 12B is a view seen from the direction perpendicular to the restricting surface, and FIG. 12C is a side view of the restricting portion having the restricting surface. In addition, the restricting portion 31 is configured as a member different from the distal end rigid portion 18.

In the modified example 1, the restricting surface 31B of the restricting portion 31 includes a groove and formed as the arc surface in the concave portion of the groove. On the other hand, as shown in FIGS. 12A, 12B, and 12C, in the modified example 2, the restriction portion is provided with a restricting surface 31C having a groove whose shape is in accordance with the outer circumference of the treatment instrument 30 having a thin diameter, in addition to the restricting surface 31B provided in the modified example 1.

Therefore, the restricting portion 31 includes two restricting surfaces 31 Band 31C which have different arc shapes. According to such a configuration, since the restricting portion 31 includes the restricting surface 31B and the restricting surface 31C, even when the treatment instruments 30 is used by being selected from treatment instruments having different diameters, one of the restricting surfaces contact the selected treatment instrument 30, and thereby capable of directing the protruding direction of the treatment instrument.

It is needless to say that the arc shape of each of the restricting surfaces 31B, 31C can be formed in accordance with the diameter of the treatment instrument 30 to be required. In addition, the restricting surfaces are not limited to the two restricting surfaces 31B, 31C, but grooves including two or more arc surfaces having different arc shapes may be provided respectively to configure the restricting surfaces.

Furthermore, as shown in FIGS. 13A, 13B, and 13C, for example, the restricting surfaces 31 B, 31C do not have to be arc surfaces, but may be configured as a restricting surface 31D having a V-shaped groove formed in a substantially V-shape. Note that FIGS. 13A to 13C are views each illustrating another configuration of the restricting surface. FIG. 13A is a view seen from the front direction in the longitudinal axis direction of the insertion portion 2, FIG. 13B is a view seen from the direction perpendicular to the restricting surface, and FIG. 13C is a side view of the restricting portion having the restricting surface.

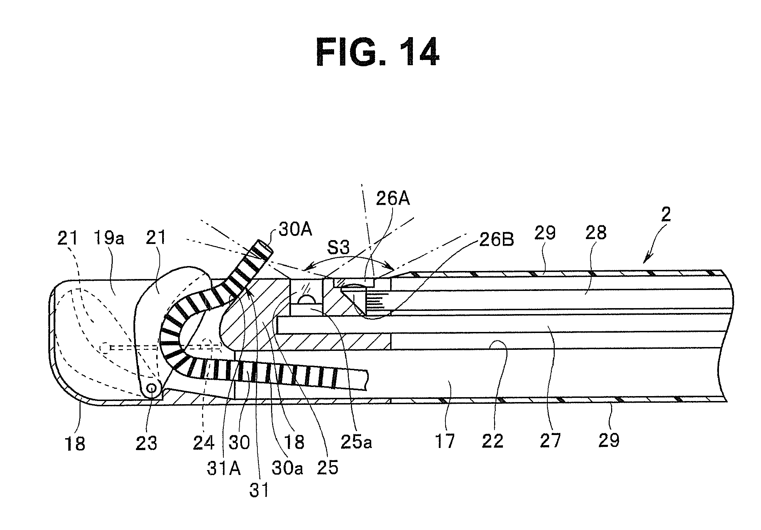

In addition, the illumination optical system of the endoscope 1 according to the present embodiment may be configured as described in modified examples 3, 4 to be described later, for example. The modified examples 3 and 4 are illustrated in FIGS. 14 to 16.

### (Modified Example 3)

FIG. 14 is a cross-sectional view of the distal end portion, which shows a configuration of an illumination optical system according to the modified example 3.

The illumination optical system according to the first embodiment irradiates the subject to be examined with the illumination light from the light guide 28 using the illumination window 26 as an optical member. On the other hand, the illumination optical system according to the modified example 3 includes an illumination window 26A configured by a diffusion lens and a prism lens 26B disposed under the illumination window.

The illumination light from the light guide 28 is incident on the prism lens 26B, and then reflected toward the illumination window 26A to be emitted. Since the illumination window 26A is configured by the diffusion lens, the illumination window diffuses the incident illumination light within the illumination range S3 shown in FIG. 14, for example, to emit the diffused light toward the subject to be examined. Note that the light irradiation range S3 of the illumination window 26A is larger than the light irradiation range S2 of the observation window 26 according to the first embodiment.

Therefore, using the illumination optical system (illumination window 26A, prism lens 26B) according to the modified example 3 enables the subject to be examined in the field-of-view range S1 of the observation window 26 to be surely and effectively irradiated with the illumination light from the light guide 28, and the illumination optical system has much larger irradiation range S3, to enable the subject to be examined in the larger range to be irradiated with the illumination light.

### (Modified Example 4)

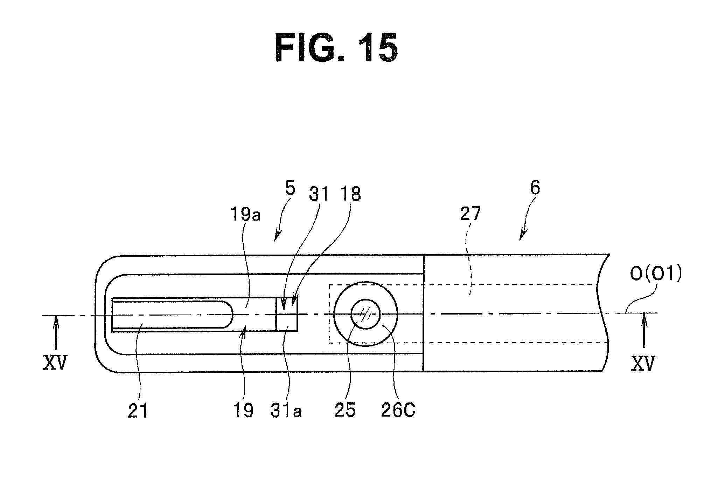

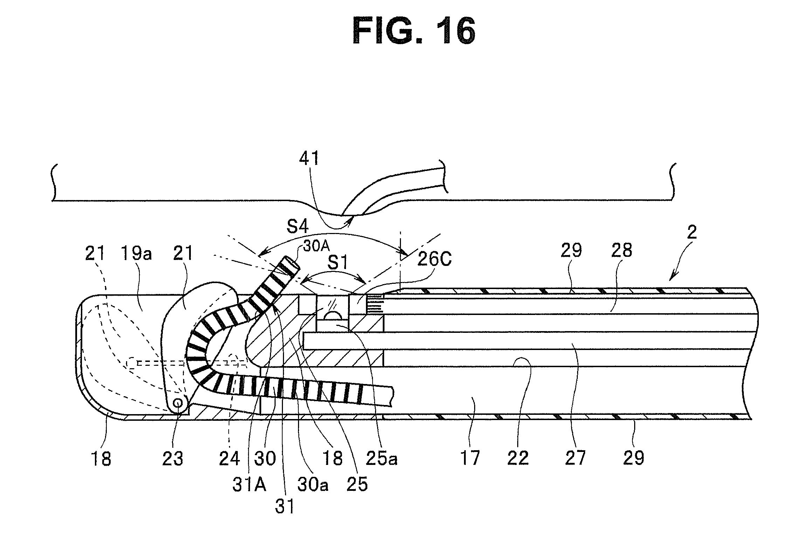

FIG. 15 is a top view of the distal end portion, which shows a configuration of an illumination optical system according to a modified example 4, and FIG. 16 is a cross-sectional view taken along the XV-XV line in FIG. 15.

The illumination optical system according to the first embodiment irradiates the subject to be examined with the illumination light from the light guide 28 using the illumination window 26 as an optical member. On the other hand, the illumination optical system according to the modified example 4 is configured by including a ring-shaped illumination window 26C formed so as to cover the outer circumference of the observation window 25.

The ring-shaped illumination window 26C includes, in the vicinity of the center thereof, a hole in which the observation window 25 is housed, and the distal end portion of the light guide 28 is arranged so as to contact the side surface of the outer circumference of the ring-shaped illumination window 26C, as shown in FIG. 16.

The ring-shaped illumination window 26C guides the illumination light from the light guide 28, which is taken in from the side surface of the illumination window, and emits the illumination light toward the subject to be examined in the light irradiation range S4 as shown in FIG. 16. Note that the diameter of the ring-shaped illumination window 26C may be appropriately changed as needed.

Therefore, using the illumination optical system (ring-shaped illumination window 26C) according to the modified example 4 is capable of irradiating the subject to be examined in the irradiation range S4 with light, with more uniform distribution of light. Furthermore, the observation window 26C can be disposed as described above, unlike the illumination window 26 provided at the rear of the observation window 25 in the first embodiment, thereby capable of reducing the length of the distal end rigid portion 18 in the longitudinal direction, which results in a size reduction of the distal end portion 5.

### (Second Embodiment)

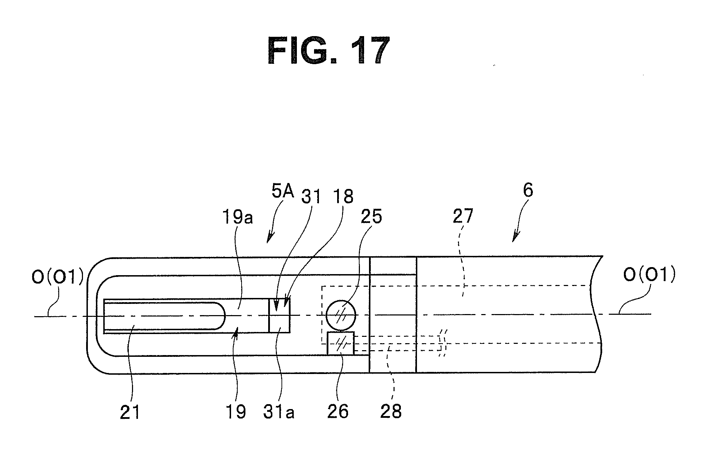

FIG. 17 is a top view of a distal end portion of an endoscope according to a second embodiment of the present invention. Note that, in FIG. 17, the same constituent elements as those in the first embodiment will be attached with the same reference numerals and descriptions thereof will be omitted, and only different parts are illustrated.

The illumination window 26 according to the first embodiment is arranged at a position closer to the operation portion in the longitudinal direction of the insertion portion 2 than the observation window 25. On the other hand, an illumination window 26 according to the second embodiment is arranged so as to be adjacent to an observation window 25 in the direction perpendicular to the longitudinal axis (central axis) O along which the insertion portion 2 is inserted.

That is, the illumination window 26 is arranged next to the observation window 25 in an observation opening portion 20. In addition, similarly as in the first embodiment, the illumination window 26 is connected with the light guide 28, to diffuse the illumination light from the light guide 28 within a predetermined irradiation range and emit the illumination light. Note that the illumination window 26 may be configured by using an optical member similar to the one used in the first embodiment, but it is desirable to configure the illumination window 26 by using an optical member such as a diffusion lens having a larger irradiation range than that of the optical member used in the first embodiment.

Other configurations and workings are the same as those in the first embodiment.

Therefore, with the second embodiment, it is possible to obtain the same effects as those in the first embodiment. In addition, arranging the illumination window 26 adjacent to the observation window 25 in the direction perpendicular to the longitudinal axis (central axis) O along which the insertion portion 2 is inserted enables reduction in length in the longitudinal direction of the distal end constituting portion 18 and in the size of the distal end portion 5.

Note that, in the first and second embodiments, description has been made on the configuration in which the illumination light from the light guide 28 is emitted using the illumination window 26, as means for obtaining illumination light, but the present invention is not limited to the configuration. For example, illumination light with which the subject to be examined may be obtained through the illumination window 26, by providing a light emitter such as an LED under the illumination window 26, connecting a driving signal line to the light emitter, and supplying a driving signal to the light emitter through the driving signal line.

In addition, when the endoscope 1 according to the first and second embodiments is connected to a predetermined video processor, marks showing the distal end position of the treatment instrument 30 according to the raised state of the treatment instrument raising base 21, the insertion direction and insertion distance of the treatment instrument 30, and the like may be displayed on the screen.

The present invention is not limited to the above-described first and second embodiments and the modified examples 1 to 4, and various changes and modifications are possible without departing from the gist of the present invention.

The present application is filed claiming the priority of Japanese Patent Application No. 2013-047117 filed in Japan on March 8, 2013, and the above described disclosure is incorporated by reference in the present description, claims and drawings.

## Claims

1. An endoscope comprising:
an operation portion;
an insertion portion to be inserted in a lumen, the insertion portion being connected to the operation portion;
a treatment instrument insertion channel through which an elongated treatment instrument is inserted, the treatment instrument insertion channel opening at a distal end portion of the insertion portion and being extended in the insertion portion from a proximal end side of the insertion portion to a part where the treatment insertion channel opens;
a treatment instrument raising base pivotally supported to be rotatable with respect to the distal end portion of the insertion portion, configured to control an inclination angle by an operation of the operation portion, and arranged to face the part where the treatment instrument insertion channel opens at the distal end portion of the insertion portion;
an objective optical system configured to obtain an image of a subject to be examined in the lumen and arranged closer to the operation portion than the treatment instrument raising base in a longitudinal direction of the insertion portion at the distal end portion of the insertion portion; and
a restricting portion configured to contact the treatment instrument which is protruded by being directed by the treatment instrument raising base when the treatment instrument raising base is inclined and to restrict an angle of the treatment instrument protruding outside the distal end portion of the insertion portion,
wherein a line connecting the treatment instrument raising base and the objective optical system is substantially parallel to a longitudinal axis in a direction in which the insertion portion is inserted, and
the restricting portion is provided so as to be located on a line segment connecting the treatment instrument raising base and the objective optical system when the objective optical system is viewed from right in front.

2. The endoscope according to claim 1, wherein the objective optical system is configured such that an optical axis of the objective optical system is directed in a direction which is not parallel to the longitudinal axis in the direction in which the insertion portion is inserted, and the restricting portion directs the treatment instrument at an angle at which an outer surface of the treatment instrument does not obstruct a distal end field of view toward which the treatment instrument is extended in a field of view of the objective optical system.

3. The endoscope according to claim 1, wherein the restricting portion restricts the angle of the treatment instrument by a restricting surface which has a predetermined area and contacts the treatment instrument.

4. The endoscope according to claim 3, wherein the restricting surface includes a groove for guiding the treatment instrument which is directed by the inclined treatment instrument raising base in a direction substantially parallel to the longitudinal axis in the direction in which the insertion portion is inserted.

5. The endoscope according to claim 3, wherein, when the treatment instrument raising base is maximally inclined, an angle of a central axis of the treatment instrument with respect to the longitudinal axis in the direction in which the insertion portion is inserted becomes equal to or smaller than an angle of the restricting surface with respect to the longitudinal axis.

6. The endoscope according to claim 1, wherein
an illumination window that illuminates the subject to be examined in the lumen by emitting illumination light guided from a light emitter is provided at the distal end portion of the insertion portion, and
the illumination window is arranged closer to the operation portion in a longitudinal direction of the insertion portion than the objective optical system.

7. The endoscope according to claim 6, wherein the illumination window is provided such that a center of the illumination window overlaps on the straight line connecting the treatment instrument raising base and the objective optical system when the illumination window is viewed from right in front.

8. The endoscope according to claim 1, wherein
an illumination window that illuminates the subject to be examined in the lumen by emitting illumination light guided from a light emitter is provided at the distal end portion of the insertion portion, and
the illumination window is arranged adjacent to the objective optical system in a direction perpendicular to the longitudinal axis along which the insertion portion is inserted.
